(19) Europäisches Patentamt
European Patent Office
Office européen des brevets

(11) **EP 0 825 174 B1**

(12) **EUROPEAN PATENT SPECIFICATION**

(45) Date of publication and mention
of the grant of the patent:
**07.11.2001 Bulletin 2001/45**

(51) Int Cl.$^7$: **C07C 209/48**

(21) Application number: **97114123.9**

(22) Date of filing: **14.08.1997**

(54) **Process for preparation of primary amine**

Verfahren zur Herstellung primärer Amine

Procédé de préparation d'amines primaires

(84) Designated Contracting States:
**DE ES FR GB**

(30) Priority: **22.08.1996 JP 22082896**

(43) Date of publication of application:
**25.02.1998 Bulletin 1998/09**

(73) Proprietor: **KAO CORPORATION**
**Chuo-ku, Tokyo (JP)**

(72) Inventors:
• **Yoshida, Wataru, c/o Kao Corporation**
**Wakayama-shi, Wakayama (JP)**
• **Fukushima, Tetsuaki, c/o Kao Corporation**
**Wakayama-shi, Wakayama (JP)**
• **Abe, Hiroshi, c/o Kao Corporation**
**Wakayama-shi, Wakayama (JP)**

(74) Representative:
**Kindler, Matthias, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 372 544          GB-A- 759 291**
**GB-A- 1 123 683          US-A- 5 583 260**

Remarks:
The file contains technical information submitted
after the application was filed and not included in this
specification

## Description

The present invention relates to a process for preparation of a primary amine. More particularly, the present invention relates to a process for preparation of a primary amine of such a high quality that no sediment may appear in the product mixture which has been derived from the primary amine.

## Background Art

Primary amines are important intermediates for household and industrial applications. Particularly, derivatives of aliphatic primary amines are finding extensive utility in such applications as fiber flexibilizing agents, antistatic agents, gasoline additives, shampoos, rinses, fungicides, and detergents.

Heretofore, as a means to produce an aliphatic primary amine, a method which comprises hydrogenating an aliphatic nitrile compound in the presence of a hydrogenating catalyst such as Raney nickel or Raney cobalt by the use of a reaction auxiliary such as an alkali like an alkali metal hydroxide or ammonium has been known (JP-B 38-21353).

However, the primary amine which is obtained by the above method, is not desired because derivative products of the primary amine such as its ethylene oxide adducts and amide compounds include sediments.

EP-A-0 659 733 refers to a method for controlling the cis/trans isomer ratio of 3-aminomethyl-3,5,5-trimethylcyclohexylamine by its manufacture from 3-cyano-3,5,5-trimethylcyclohexanone by aminating hydrogenation, whereby isophoronitrile in the presence of ammonia and a suspension or fixed bed hydrogenation catalyst is reacted with hydrogen at a pressure of 3-20 mPa at a temperature up to 150°C, wherein the hydrogenation in a first step is effected at 10-90°C and in a following second step at 90-150°C, wherein the temperature difference between the first and the second step is at least 30°C.

GB-A-1 123 683 refers to a continuous process for the manufacture of hexamethylene diamine by hydrogenation of adiponitrile, wherein the hydrogenation is performed under pressure and under conditions such that the temperature of the reaction mixture leaving the hydrogenation zone is at least 120°C. Preferably, the hydrogenation is performed at a starting temperature of 80-100°C to a finishing temperature of 120-150°C.

In GB-A-759 291 a process for the production of saturated secondary amines is described which comprises preheating a mixture of a starting material consisting of an aliphatic nitrile, a primary aliphatic amine or a mixture of such nitrile and such amine, and a hydrogenation catalyst to remove substantially all of the moisture therefrom, contacting said mixture under elevated pressure with hydrogen, wherein the mixture is preheated to a temperature of from 120-150°C and wherein the contacting step is carried out at a pressure of from 100-500 psig and a temperature of from 130-160°C.

## Disclosure of the Invention

An object of the present invention, therefore, is to provide a process for preparation of a primary amine of such a high quality that no sediment may appear in the product mixture which has been derived from the primary amine.

The present inventors have made a diligent study with a view to attaining the object mentioned above and have consequently found that this object is accomplished by hydrogenating an aliphatic nitrile compound under specific reaction conditions. The present invention has been perfected as a result.

Specifically, the present invention provides a process for preparation of a primary amine, which comprises the step of catalytically hydrogenating an aliphatic nitrile compound in the presence of a hydrogenating catalyst under the following conditions (A) to (C).

Condition (A):

$$(T_{max} - T_{min}) \geqq 10°C$$

wherein $T_{max}$ represents the highest temperature of the reaction temperature T (°C) in the range of $10 \leqq X \leqq 100$; $T_{min}$ represents the lowest temperature of the reaction temperature T (°C) in the range of $10 \leqq X \leqq 100$; X represents the conversion (%) based on the aliphatic nitrile compound.

Condition (B):

$$(0.5X + 70) \leqq T \leqq (0.5X + 100)$$

In the range of $10 \leqq X \leqq 100$ wherein T is a reaction temperature and X has the same meaning as defined above; and

Condition (C):
the reaction temperature T is elevated at a rate of at least 1.4°C/min. until it reaches 75°C.

Now, the mode of embodying the present invention will be described in detail below.

The aliphatic nitrile compound as the raw material to be used in the present invention, though not specifically limited, is preferably linear or branched saturated or unsaturated aliphatic nitrile compounds having 8 to 36 carbon atoms, more preferably Saturated aliphatic nitrile compounds, and particularly preferably linear saturated aliphatic nitrile compounds having 8 to 18 carbon atoms.

As concrete examples of the aliphatic nitrile compound, caprylonitrile, caprinitrile, lauronitrile, coconut aliphatic nitrile, tallow aliphatic nitrile, stearonitrile, oleonitrile, linolonitrile, linoleonitrile, ergonitrile, behenonitrile, and nitrile compounds having a branched chain(s) derived from synthetic branched chain-containing fatty acids produced by the oxo method and the Guerbet synthetic method, and mixtures thereof, may be cited.

As concrete examples of the hydrogenating catalyst to be used in the present invention, Raney cobalt catalysts, Raney nickel catalyst, platinum, ruthenium, rhodium, and palladium. These hydrogenating catalysts may be used either singly or in the form of a mixture of two or more members. It is particularly advantageous to use at least one member selected from the group consisting of Raney cobalt catalysts and Raney nickel catalysts. The Raney cobalt catalysts include Raney cobalt and Raney cobalt·manganese and the Raney nickel catalysts include Raney nickel and Raney nickel·manganese. These are obtained by developing alloys of the relevant metals with aluminum by the standard method using an alkali.

The hydrogenating catalyst may be in any of the forms in conventional use. The simple form, oxides, hydroxides and other various salts of these metals mentioned above, and mixtures thereof may be used as the catalyst. The metals which form the catalysts mentioned above may be deposited on proper carriers such as alumina, silica alumina, diatomaceous earth, silica, activated carbon, and natural and synthetic zeolites. Further, the hydrogenating catalyst may be the salt of an aliphatic carboxylic acid with a catalyst-forming metal or the complex of the catalyst-forming metal stabilized with a proper ligand. The various catalysts mentioned above, when necessary, may be used as suitably mixed.

The amount of the hydrogenating catalyst to be used in the process of preparation of the present invention, though not specifically limited, is generally in the range of 0.05 to 10% by weight based on the amount of the nitrile compound as the raw material.

The process of preparation of the present invention requires the reaction to proceed at such a temperature as to satisfy the condition (A) and the condition (B) mentioned above.

To be specific, the difference between the highest value and the lowest value of the reaction temperature in the range of 10 to 100% of the conversion X based on the aliphatic nitrile compound is not less than 10°C, preferably in the range of 15 to 75°C, and particularly in the range of 20 to 40°C. The reaction temperature T in the range of 10 to 100% of the conversion X is not less than $(0.5X+70°C)$ and not more than $(0.5X+100°C)$.

If the elevation of temperature is so carried out as not to satisfy the condition (A) mentioned above, the primary amine of high quality aimed at by the present invention will not be obtained.

According to the invention the reaction temperature is elevated at a rate of not less than 1.4°C/minute until it reaches 75°C.

Further, the present invention is preferred to elevate the reaction temperature T continuously and/or stepwise. By elevating the reaction temperature in this manner, the primary amine of still higher quality, i.e. such a high quality that no sediment may appear in the product mixture which has been derived from the primary amine, can be obtained.

In the process of preparation of the present invention, the reaction is preferred to proceed in the presence of the hydroxide of an alkali metal or alkaline earth metal, the alcoholate of an alkali metal or alkaline earth metal, or ammonia in addition to the hydrogenating catalyst mentioned above for the purpose of improving the selectivity of the reaction. As concrete examples of the hydroxide of an alkali metal or alkaline earth metal, sodium hydroxide, potassium hydroxide, and calcium hydroxide may be cited. As concrete examples of the alcoholate of an alkali metal or alkaline earth metal, sodium methylate, sodium ethylate, potassium methylate, potassium ethylate, calcium methylate, and calcium ethylate may be cited. Among them, the hydroxides of alkali metals and ammonia prove particularly appropriate.

The amount of an alkali or ammonia to be used, for the purpose of securing the selectivity fully satisfactorily and avoiding to degrade the catalytic activity, is preferred to be in the range of 0.05 to 1.0% by weight based on the amount of the nitrile compound as the raw material.

The hydrogenating reaction of the present invention, when performed in the presence of a prescribed amount of water, is enabled to improve the hydrogenating reaction velocity thereof without inhibiting the high selectivity thereof. The amount of the water to be used is preferably 0.005 to 1 time, particularly preferably 0.01 to 0.5 time, that of the aliphatic nitrile compound as the raw material.

Now, a preferred mode of embodying the process of preparation of the present invention will be described below.

A pressure resistance reaction vessel provided with a hydrogen inlet tube and a tube for sampling is charged with an aliphatic nitrile compound as the raw material, a hydrogenating catalyst, and an alkali such as the hydroxide of an alkali metal. Hydrogen is introduced into the vessel until a prescribed pressure. This pressure of hydrogen is in the range of from 0 MPa (gauge) to 5 MPa (gauge) preferably 0.5 to 2.0 MPa (gauge). Then, the temperature of the reaction vessel is elevated. At this time, the elevation of the temperature is carried out so as to satisfy the conditions (A), (B) and (C).

After the reaction is completed, the reaction product is directly distilled or filtered to be separated into the reaction product and the catalyst.

Brief Description of the Drawings

Fig. 1 is a diagram showing the relation between the conversion and the reaction temperature in Example 1.

Fig. 2 is a diagram showing the relation between the conversion and the reaction temperature in Example 9.

Fig. 3 is a diagram showing the relation between the conversion and the reaction temperature in Example 10.

Examples

The present invention will be described more specifically below with reference to the working examples to be cited herein below. It should be noted, however, that the present invention is not limited to these working examples.

Example 1

In an autoclave having an inner volume of 1 liter, 300 g of stearonitrile, 0.9 g of developed Raney nickel catalyst (alloy composition before development Ni : Al = 40.2 : 59.8), 6 g of water, and 0.3 g of sodium hydroxide were placed, pressed with hydrogen gas to 2.0 MPa (gauge), and heated at a rate of 1.4°C/minute up to 100°C. The conversion (of the nitrile compound as the raw material) reached 10% while the reaction is proceeding at 100°C. The reaction temperature was elevated to 120°C when the conversion reached 40% and further to 130°C when the conversion reached 70%. The absorption of hydrogen ceased in 3.0 hours. The condition was kept intact for 30 minutes to complete the reaction. The crude reaction product was purified by distillation to obtain stearyl amine. The stearyl amine produced no suspension of sediment.

1 mole of ethylene oxide was added to the obtained stearyl amine at 160°C and then one more mole was added at 120°C. This way 2 moles were added in total. When the adduct was placed in a beaker, 100 ml in inner volume, and visually examined at room temperature, it showed absolutely no sign of suspension of a sediment.

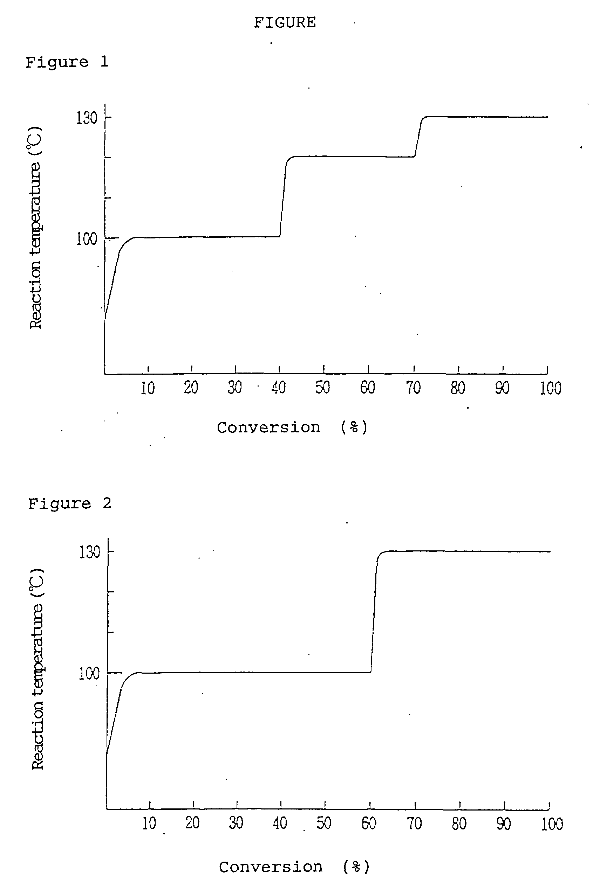

The relation between the conversion and the reaction temperature obtained herein is shown in Fig. 1.

Example 2

Stearyl amine was obtained by following the procedure of Example 1 while charging the reaction vessel with 1.5 g of the developed Raney nickel catalyst, 15 g of water, and 0.6 g of sodium hydroxide. In this case, the absorption of hydrogen lasted for 1.7 hours and the reaction product formed no suspension of a sediment.

When the produced stearyl amine was subjected to the same addition of ethylene oxide as in Example 1, the adduct formed no suspension of a sediment.

Example 3

In an autoclave having an inner volume of 1 liter, 300 g of stearonitrile and 0.9 g of a developed Raney nickel catalyst were placed, pressed with ammonia gas to 0.2 MPa (gauge), further pressed with hydrogen gas to 2.0 MPa (gauge), and heated at the same temperature as in Example 1 to induce a reaction and obtain stearyl amine. At this time, the absorption of hydrogen lasted for 4.2 hours. The reaction product formed no suspension of a sediment.

When the produced stearyl amine was subjected to the same addition of ethylene oxide as in Example 1, the adduct formed no suspension of a sediment.

Comparative Example 1

Stearyl amine was obtained by following the procedure of Example 1 while keeping the reaction temperature at 130°C from the start through the completion of the reaction. At this time, the absorption of hydrogen lasted for 2.1 hours. The reaction product formed no suspension of a sediment.

When the produced stearyl amine was subjected to the same addition of ethylene oxide as in Example 1, the adduct gave rise to suspension of a sediment.

Comparative Example 2

Stearyl amine was obtained by following the procedure of Example 1 while keeping the reaction temperature at 110°C from the start through the completion of the reaction. At this time, since the absorption of hydrogen continued after the elapse of 10 hours, the reaction was stopped in 10 hours (the conversion was 85%). The product obtained after the stop of the reaction was refined by distillation to obtain stearyl amine. This stearyl amine produced no suspension of a sediment.

When the produced stearyl amine was subjected to the same addition of ethylene oxide as in Example 1, the adduct gave rise to suspension of a sediment.

Comparative Example 3

Stearyl amine was obtained by following the procedure of Example 1 while performing the elevation of temperature at a rate of 1.2°C/minute to 100°C. At this time, the absorption of hydrogen lasted for 3.1 hours. The reaction product formed no suspension of a sediment.

When the produced stearyl amine was subjected to the same addition of ethylene oxide as in Example 1, the adduct gave rise to slight suspension of a sediment.

Example 4

A primary amine was obtained by following the procedure of Example 1 while using Guerbet nitrile (having 36 carbon atoms) instead as the raw material. At this time, the absorption of hydrogen lasted for 4.2 hours. The reaction product formed no suspension of a sediment.

When the produced primary amine was subjected to the same addition of ethylene oxide as in Example 1, the adduct gave rise to no suspension of a sediment.

Example 5

Octyl amine was obtained by following the procedure of Example 1 while using caprylonitrile instead as the raw material. At this time, the absorption of hydrogen lasted for 4.9 hours. The reaction product formed no suspension of a sediment. When the produced octyl amine was subjected to the same addition of ethylene oxide as in Example 1, the adduct gave rise to no suspension of a sediment.

Example 6

Octadecenyl amine was obtained by following the procedure of Example 1 while using oleonitrile instead as the raw material. At this time, the absorption of hydrogen lasted for 5.2 hours. The reaction product formed no suspension of a sediment.

When the produced octadecenyl amine was subjected to the same addition of ethylene oxide as in Example 1, the adduct gave rise to no suspension of a sediment.

Example 7

Stearyl amine was obtained by following the procedure of Example 1 while using a developed Raney cobalt catalyst (alloy composition before development Co : Al = 49.6 : 50.4) instead as the catalyst. At this time, the absorption of hydrogen lasted for 4.8 hours. The reaction product formed no suspension of a sediment.

When the produced stearyl amine was subjected to the same addition of ethylene oxide as in Example 1, the adduct gave rise to no suspension of a sediment.

Example 8

Stearyl amine was obtained by following the procedure of Example 1 while changing the reaction temperature so that the relation between the conversion and the reaction temperature might equal the graph of Fig. 2. At this time, the absorption of hydrogen lasted for 4.2 hours. The reaction product formed no suspension of a sediment.

When the produced stearyl amine was subjected to the same addition of ethylene oxide as in Example 1, the adduct gave rise to no suspension of a sediment.

Example 9

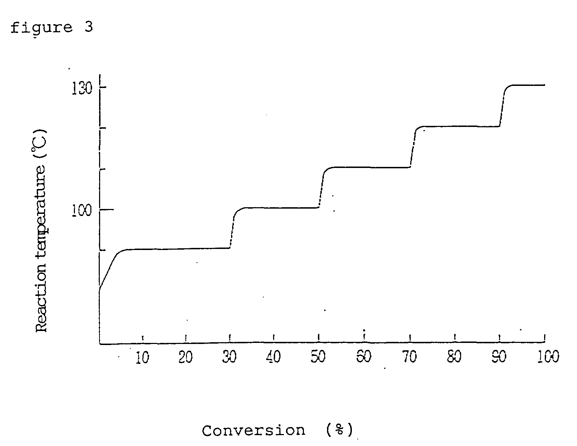

Stearyl amine was obtained by following the procedure of Example 1 while changing the reaction temperature so that the relation between the conversion and the reaction temperature might equal the graph of Fig. 3. At this time, the absorption of hydrogen lasted for 6.1 hours. The reaction product formed no suspension of a sediment.

When the produced stearyl amine was subjected to the same addition of ethylene oxide as in Example 1, the adduct gave rise to no suspension of a sediment.

**Claims**

1. A process for preparation of a primary amine, which comprises the step of catalytically hydrogenating an aliphatic nitrile compound in the presence of a hydrogenating catalyst under the following conditions (A) to (C):

   Condition (A):

   $$(T_{max} - T_{min}) \geq 10°C$$

   wherein $T_{max}$ represents the maximum of the reaction temperature T (°C) in the range of $10 \leq X \leq 100$; $T_{min}$ represents the minimum of the reaction temperature T (°C) in the range of $10 \leq X \leq 100$; X represents the conversion (%) based on the aliphatic nitrile compound;
   Condition (B):

   $$(0.5X + 70) \leq T \leq (0.5X + 100)$$

   in the range of $10 \leq X \leq 100$ wherein T and X have the same meaning as defined above; and
   Condition (C):
       the reaction temperature T is elevated at a rate of at least 1.4°C/min. until it reaches 75°C.

2. The process according to Claim 1, wherein the reaction temperature T is elevated continuously and/ or stepwise.

3. The process according to Claim 1 or 2, wherein the aliphatic nitrile compound is a linear or branched, saturated or unsaturated, aliphatic nitrile compound having 8 to 36 carbon atoms.

4. The process according any of Claims 1 to 3, wherein the hydrogenating catalyst is at least one member selected from the group consisting of Raney cobalt catalysts and Raney nickel catalysts.

5. The process according to Claim 1, wherein $75°C \geqq (T_{max} - T_{min}) \geqq 15°C$.

6. The process according to Claim 1, wherein $40°C \geqq (T_{max} - T_{min}) \geqq 20°C$.

7. The process according to Claim 1, wherein the hydrogenation is carried out further in the presence of a hydroxide of an alkali metal or an alkaline earth metal, an alcoholate of an alkali metal or an alkaline earth metal, or ammonia.

8. The process according to Claim 1, wherein 0.05 to 10% by weight of the hydrogenating catalyst based on the aliphatic nitrile compound is used.

9. The process according to Claim 1, wherein the hydrogenation is carried out in the presence of water.

**Patentansprüche**

1. Verfahren zur Herstellung eines primären Amins, umfassend die katalytische Hydrierung einer aliphatischen Nitril-Verbindung in der Gegenwart eines Hydrierungskatalysators unter den folgenden Bedingungen (A) bis (C):

   Bedingung (A):

   $$(T_{max} - T_{min}) \geq 10°C$$

   worin $T_{max}$ das Maximum der Reaktionstemperatur T (°C) im Bereich von $10 \leq X \leq 100$ ist; $T_{min}$ das Minimum der Reaktionstemperatur T (°C) im Bereich von $10 \leq X \leq 100$ ist; X die Umwandlung (%) ist, bezogen auf die aliphatische Nitril-Verbindung;
   Bedingung (B):

   $$(0,5X + 70) \leq T \leq (0,5X + 100)$$

   in dem Bereich von $10 \leq X \leq 100$, worin T und X die gleichen Bedeutungen wie oben definiert aufweisen; und
   Bedingung (C):
   die Reaktionstemperatur T wird bei einer Rate von wenigstens 1,4°C/min erhöht, bis sie 75°C erreicht.

2. Verfahren nach Anspruch 1, worin die Reaktionstemperatur T kontinuierlich und/oder schrittweise erhöht wird.

3. Verfahren nach Anspruch 1 oder 2, worin die aliphatische Nitril-Verbindung eine lineare oder verzweigte, gesättigte oder ungesättigte, aliphatische Nitril-Verbindung mit 8 bis 36 Kohlenstoffatomen ist.

4. Verfahren nach einem der Ansprüche 1 bis 3, worin der Hydrierungskatalysator zumindest eine Verbindung ist, ausgewählt aus der Gruppe, bestehend aus Raney-Cobalt-Katalysatoren und Raney-Nikkel-Katalysatoren.

5. Verfahren nach Anspruch 1, worin $75°C \geqq (T_{max} - T_{min}) \geqq 15°C$.

6. Verfahren nach Anspruch 1, worin $40°C \geqq (T_{max} - T_{min}) \geqq 20°C$.

7. Verfahren nach Anspruch 1, worin die Hydrierung weiter in der Gegenwart eines Hydroxides eines Alkalimetalls oder Erdalkalimetalls, eines Alkoholates eines Alkalimetalls oder Erdalkalimetalls oder Ammoniak durchgeführt wird.

8. Verfahren nach Anspruch 1, worin 0,05 bis 10 Gew.-% des Hydrierungskatalysators verwendet werden, bezogen auf die aliphatische Nitril-Verbindung.

9. Verfahren nach Anspruch 1, worin die Hydrierung in der Gegenwart von Wasser durchgeführt wird.

**Revendications**

1. Procédé de préparation d'une amine primaire, qui comprend une étape consistant à hydrogéner catalytiquement un composé nitrile aliphatique en présence d'un catalyseur d'hydrogénation dans les conditions (A) à (C) suivantes :

   Condition (A) :

   $$(T_{max} - T_{min}) \geq 10 °C$$

   où $T_{max}$ représente le maximum de la température de réaction T (°C) dans la plage de $10 \leq X \leq 100$ ; $T_{min}$ représente le minimum de la température de réaction T (°C) dans la plage $10 \leq X \leq 100$ ; X représente la conversion (%) sur la base du composé nitrile aliphatique :
   Condition (B) :

   $$(0,5X + 70) \leq T \leq (0,5X + 100)$$

   dans la plage de $10 \leq X \leq 100$ où T et X ont la même signification que celle donnée ci-dessus ; et

Condition (C) :

la température de réaction T est augmentée à une vitesse d'au moins 1,4 °C / min. jusqu'à ce qu'elle atteigne 75 °C.

2. Procédé selon la revendication 1, dans lequel la température de réaction T est augmentée de manière continue et / ou par étapes.

3. Procédé selon la revendication 1 ou la revendication 2, dans lequel le composé nitrile aliphatique est un composé nitrile aliphatique linéaire ou ramifié, saturé ou insaturé ayant de 8 à 36 atomes de carbone.

4. Procédé selon l'une quelconque des revendications 1 à 3, dans lequel le catalyseur d'hydrogénation est au moins un élément choisi dans le groupe constitué par les catalyseurs de cobalt de Raney et les catalyseurs de nickel de Raney.

5. Procédé selon la revendication 1, dans lequel 75 °C $\geq$ ($T_{max}$ - $T_{min}$) $\geq$ 15 °C.

6. Procédé selon la revendication 1, dans lequel 40 °C $\geq$ ($T_{max}$ - $T_{min}$) $\geq$ 20 °C.

7. Procédé selon la revendication 1, dans lequel l'hydrogénation s'effectue, en outre, en présence d'un hydroxyde d'un métal alcalin ou d'un métal alcalino-terreux, d'un alcoolate d'un métal alcalin ou d'un métal alcalino-terreux, ou d'ammoniac.

8. Procédé selon la revendication 1, dans lequel on utilise de 0,05 à 10 % en poids d'un catalyseur d'hydrogénation basé sur le composé nitrile aliphatique.

9. Procédé selon la revendication 1, dans lequel l'hydrogénation s'effectue en présence d'eau.

FIGURE

Figure 1

Figure 2

figure 3

Conversion (%)